# EUROPÄISCHE PATENTSCHRIFT

(11) **EP 3 981 357 B1**
(45) Veröffentlichungstag und Bekanntmachung des Hinweises auf die Patenterteilung: **26.07.2023**
(21) Anmeldenummer: 20200962.7
(22) Anmeldetag: 09.10.2020
(51) Int. Cl.: A61C 5/90

(54) **FOLIENSPANNELEMENT**
FILM CLAMPING ELEMENT
ÉLÉMENT TENDEUR DE FILM

(43) Veröffentlichungstag der Anmeldung: 13.04.2022
(73) Patentinhaber: Ivoclar Vivadent AG, 9494 Schaan (LI)
(72) Erfinder: Lichtensteiger, Markus, 9462 Montlingen (CH); Pokorny, Walter, 6719 Bludesch (AT); Müller, Frank, 6800 Feldkirch (AT)
(74) Vertreter: Baldus, Oliver

(56) Entgegenhaltungen:
- WO-A1-03/051185
- DE-A1- 3 329 919

## Beschreibung

Die Erfindung betrifft ein Folienspannelement, gemäß dem Oberbegriff von Anspruch 1.

Ein derartiges Folienspannelement wird seit über zehn Jahren unter dem Namen OPTRAGATE erfolgreich eingesetzt, um den freien Zugang zum Patientenmund bei dentalen Bearbeitungen im Mundraum eines Patienten zu ermöglichen. Es besteht aus einem Vestibulärring und einem Lippenring, zwischen denen sich eine Folie erstreckt. Bei diesem Element ist die Folie gegenüber beiden Ringen verschieblich gelagert und elastisch. Zur Vermeidung von allergischen Beeinträchtigungen ist sie latexfrei ausgebildet.

Die Druckschrift WO 03/051185 A1 betrifft einen Lippen- und Wangenexpander zur Verwendung beim Ausführen dentalmedizinischer, dentalhygienischer oder dentaldokumentierender Tätigkeiten.

Die Druckschrift DE 33 29 919 A1 betrifft einen Lippenschutz zum Einsatz während einer ärztlichen, chirurgischen Behandlung der Zähne und/oder des Mund- oder Rachenraumes.

Ferner ist aus der EP 3 666 221 A1 ein Folienspannelement bekannt, bei dem ein Elastikband in die Folie integriert ist. Das Elastikband doppelt die Folie auf, so dass dort also der Folie alleine, eine größere Spannkraft hat. Durch den radial einwärts gewandten Zug des Elastikbandes auf die Folie ergibt sich eine Einschnürung - sowohl des Elastikbandes als auch der Folie -, so dass der radial auswärts weisende Druck auf die Lippen reduziert ist.

Diese Ausführungsform hat im Vergleich mit den übrigen in der genannten Druckschrift beschriebenen Ausführungsformen den Nachteil, dass sie vergleichsweise aufwändig und teuer in der Herstellung ist. Zudem bewirkt das umgeschlagene Elastikband eine permanente Spannung innerhalb der Folie und des Bandes. Während der Lagerungsdauer kann dieser Einschnürungseffekt erschlaffen und seine Funktion verlieren.

Zwar wäre die Integration des Elastikbandes in die Folie, beispielsweise durch Umschweißen, möglich. Die radial einwärts gewandte Spannkraft führt jedoch zur Faltenbildung, so dass sich diese Lösung mit dem aufgedoppelten Elastikband nicht durchgesetzt hat, sondern eine der übrigen in der genannten Patentveröffentlichung beschriebenen Ausführungsformen.

Demgegenüber liegt der Erfindung die Aufgabe zugrunde, ein Folienspannelement gemäß dem Oberbegriff von Anspruch 1 zu schaffen, das die Tragequalität des Folienspannelements verbessert.

Diese Aufgabe wird erfindungsgemäß durch Anspruch 1 gelöst. Vorteilhafte Weiterbildungen ergeben sich aus den Unteransprüchen.

Überraschend ergibt die Kombination der erfindungsgemäßen Maßnahmen, also zum einen, die Folie selbst mit einer sich radial einwärts erstreckenden Einschnürung zu versehen, und zum anderen, die Folie spannungsfrei herzustellen, also, dass sich die Einschnürung in der Folie spannungsfrei selbst ergibt, ohne dass eine Spannkraft durch ein die Folie verdickende Elastikband realisiert werden müsste, den Vorteil, dass keine Falten entstehen, aber dennoch kein radial auswärts weisender Druck auf die Lippen ausgeübt wird.

Überraschend stellte sich zudem ein weiterer Vorteil ein, nämlich, dass eine Materialersparnis möglich ist. Die Folie kann über ihren Verlauf eine gleichbleibende Materialstärke aufweisen, und hierdurch ergibt sich eine Materialeinsparung sowohl im Vergleich mit der dargestellten Ausführungsform gemäß Figur 6 der EP 3 666 221 A1, als auch sogar gegenüber der klassischen OPTRAGATE-Lösung, bei der sich die Folie zwischen Vestibulärring und Lippenring kegelstumpfförmig erstreckt, wenn man das Folienspannelement an einem der Ringe hält.

Das Folienspannelement ist ein Massenprodukt. Großtechnisch betrachtet fällt daher diese Materialeinsparung ins Gewicht und erlaubt eine deutliche Verminderung der Herstellkosten.

Die Folie erstreckt sich in an sich bekannter Weise ringförmig oder schlauchförmig zwischen dem Lippenring und dem Vestibulärring.
In vorteilhafter Ausgestaltung ist es vorgesehen, die Einschnürung lediglich teilumlaufend vorzusehen. Bekanntlich hat ein halb geöffneter Mund im Wesentlichen eine ovale Form. Dieser Form kann nun Rechnung getragen werden, indem die Einschnürung mittig ist, also beispielsweise sich über einen oberen und einen unteren Viertelkreis bei in den Mund eingesetztem Folienspannelement erstreckt und im Bereich der beiden äußeren Viertelkreise keine Einschnürung vorgesehen ist. Hierdurch lässt sich eine besondere scanfreundliche Ausgestaltung realisieren, die es auch ermöglicht, seitliche Bereiche des Mundes des Patienten zu scannen.

Die Materialstärke der Folie ist bevorzugt über die gesamte Folie konstant.

Die Materialstärke kann beispielsweise zwischen 0,1 mm und 1mm betragen und beträgt bevorzugt zwischen 0,3 und 0,4mm. Die Materialstärke der Folie eines Folienspannelements, mit denen Kinder versorgt werden, beträgt bevorzugt etwas weniger, beispielsweise 0,2 oder 0,3mm. Hierdurch ist die Spannkraft der Folie vermindert, und überraschend ergeben sich auch bei einer solchen, kleineren Ausgestaltung des erfindungsgemäßen Folienspannelements keine Falten, oder höchstens ganz geringe Falten.

Erfindungsgemäß ist es auch besonders günstig, dass sich die Stelle, an welcher die Einschnürung realisiert ist, in weiten Bereichen an die Erfordernisse anpassen lässt. In bevorzugter Ausgestaltung erstreckt sich die Einschnürung dem Vestibulärring stärker benachbart als dem Lippenring. Hierdurch ist es möglich, den Vestibulärring mit einem vergleichsweise geringen Durchmesser auszugestalten, was wiederum zu einer Materialersparnis führt. Die Durchmesserreduktion kann beispielsweise zwischen 10 und 20 Prozent betragen.

In einer anderen Ausgestaltung ist die Einschnürung nahe dem Lippenring vorgesehen.

Dadurch, dass die Folie frei von Auflagen und/oder Einlagen ist, ist ihre Spannkraft bei Belastung über ihre gesamte Erstreckung konstant. Dies gilt sowohl in longitudinaler Richtung, also in der Richtung zwischen Vestibulärring und Lippenring, als auch in tangentialer Richtung, also umlaufend.

Dies führt auch dazu, dass eingeleitete Spannungen gleichmäßig über die gesamte Erstreckung aufgenommen werden. Hierdurch wird die Folie durch die eingeleitete radial einwärts wirkende Spannung gleichmäßig belastet. Die Belastung pro Flächeneinheit ist damit deutlich geringer, als wenn ein zusätzliches Elastikband mit der Folie intensiv verbunden ist.

Die in vorteilhafter Ausgestaltung vorgesehene konstante Stärke hat insofern den besonderen Vorteil, dass die Reißfestigkeit im Vergleich zur beschriebenen vorbekannten Lösung steigt. Auch mit dieser Maßnahme ist es möglich, die Herstellkosten zu reduzieren, denn trotz gleicher Größe bei gleichem Materialgewicht ist die Lösung mit der Verdickung weniger reißfest als die erfindungsgemäße Lösung.

Während eine Schwankung der Materialstärke von beispielsweise 10 Prozent, was die Stabilität der Folie angeht nicht ins Gewicht fällt, wird durch eine signifikante, als Elastikband ausgebildete, Verdickung, die sich über beispielsweise ein Drittel der Länge der Folie in longitudinaler Richtung erstreckt, der übrige Teil der Folie geschwächt; die Dehnung der Folie ergibt sich praktisch ausschließlich an den Übrigen zwei Dritteln der Folie, zumindest dann, wenn die Materialstärke der Verdickung beispielsweise durch die integrierte Elastikeinlage beispielsweise das Dreifache der Folienstärke im Übrigen beträgt.

Insofern beträgt die Reißfestigkeit des Folienspannelements der vorbekannten Lösung trotz höherem Gesamtgewicht nur etwa zwei Drittel der Reißfestigkeit des erfindungsgemäßen Folienspannelements.

Die Tiefe der Einschnürung lässt sich in weiten Bereichen an die Erfordernisse anpassen. So kann die Tiefe gleichsam auch einen ovalen Verlauf haben. In einer ersten Ausgestaltung insofern ist sie im Mundmittenbereich am größten und im Seitenbereich am geringsten.

In einer zweiten Ausgestaltung sind diese Verhältnisse umgekehrt.

Es ist auch möglich, die Tiefe der Einschnürung in tangentialer Richtung betrachtet von vornherein unterschiedlich vorzusehen, mit sanften Übergängen zwischen den unterschiedlichen Tiefen, und beim Einsatz des Folienspannelements am und im Mund des Patienten das Folienspannelement so zu drehen, dass der Tragekomfort so am besten ist. Aufgrund der unterschiedlichen Ausgestaltungen von Patientenmündern lässt sich insofern mit dieser Maßnahme eine optimierte patientenspezifische Anpassung erzielen.

Die Herstellung des erfindungsgemäßen Folienspannelements kann in beliebiger Weise erfolgen. Beispielsweise lässt sich die Folie durch einen Spritzgießprozess herstellen, wobei die Spritzgussform die Ausgestaltung der späteren spannungsfreien Form der Folie im biegeschlaffen Zustand vorgibt. Die Ringe können in die Spritzgussform eingelegt sein und umspritzt werden.

Anstelle dessen ist auch ein Tauchprozess oder ein Blasformprozess möglich.

Es ist auch möglich, in besonderen Fällen die Folie um ihren Umfang betrachtet mit unterschiedlichen Stärken zu versehen, wobei bevorzugt auch hier die Unterschiede nicht mehr als 20 Prozent betragen und wiederum sanfte Übergänge zwischen den unterschiedlichen Stärken der Folien vorgesehen sind.

Wichtig für die zuvor beschriebene besonders gute Reißfestigkeit der Folie ist es, dass die Folie in longitudinaler Richtung betrachtet eine gleichbleibende Stärke aufweist. Die größte eingeleitete Spannung entsteht durch die Lippe des Patienten, die die Folie in longitudinaler Richtung spannt.

Soweit der Stärkenverlauf in longitudinaler Richtung unterschiedlich gewählt werden soll, ist es bevorzugt, auch hier die Schwankung und die Unterschiede auf +/- 20 Prozent zu beschränken.

In weiterer vorteilhafter Ausgestaltung ist eine Netzstruktur der Folie vorgesehen. Dies ist besonders bevorzugt so realisiert, dass die Maschen des Netzes eine geringere Folien-Wandstärke als das Gitter des Netzes aufweisen. Die Abdichtung durch die erfindungsgemäße Folie wird hierdurch nicht beeinträchtigt, aber die Festigkeit im Verhältnis zum eingesetzten Gewicht ist weiter verbessert.

Dies gilt insbesondere dann, wenn die Netzstruktur unter Berücksichtigung einer Berechnung nach der Finite-Elemente-Methode festgelegt wird.

Die radiale Tiefe der Einschnürung kann in weiten Bereichen an die Anforderungen angepasst werden. Sie bemisst sich als Durchmesserverminderung gegenüber einem Standard-OPTRAGATE-Folienspannelement, also dem Folienspannelement gemäß dem Stand der Technik.

Dieses erstreckt sich kegelstumpförmig, zumindest, wenn es am Lippenring oder am Vestibulärring aufgehängt gehalten wird, ohne dass eine zusätzliche Spannung und/oder Zugkräfte eingeleitet würden.

Wenn das erfindungsgemäße Folienspannelement am Lippenring oder am Vestibulärring aufgehängt gehalten wird, ohne dass eine zusätzliche Spannung und/oder Zugkräfte eingeleitet würden, hat es eine Form mit einer sich radial einwärts erstreckende Einschnürung.

Die Tiefe der Einschnürung kann 5 bis 35% des Durchmessers des Lippenrings betragen, insbesondere etwa 15%.

Erfindungsgemäß besonders günstig ist es, dass die Folie sich einstückig erstreckt. Die Einstückigkeit gilt in alle Richtungen, also in achsparalleler Richtung zwischen Vestibulärring und Lippenring, in tangentialer Richtung aber auch in Richtung ihrer Dicke.

Dies bedeutet, dass die Folie zwischen ihrer radial nach einwärts weisenden Innenfläche und ihrer radial nach auswärts weisenden Außenfläche ebenfalls einstückig ist und keine Auflagen und/oder Einlagen hat.

Durch die Einstückigkeit wird ein Spannungsgefälle innerhalb der Folie verhindert oder vermindert. Die Reißfestigkeit wird hierdurch erhöht, und es entstehen an keiner Stelle Spannungsspitzen, vielmehr werden die eingeleiteten Spannungen vergleichmäßigt.

Die erfindungsgemäße Einschnürung kann auch als Konkavität oder als Einwölbung betrachtet werden. Wichtig ist die Veränderung des Durchmessers, entlang der Achse betrachtet. Vom Lippenring ausgehend nimmt der Durchmesser der Folie zunächst ab, und zwar ohne Unstetigkeitstelle und zunächst monoton fallend.

Im Bereich der tiefsten Einschnürung ist der Durchmesser der Folie am geringsten. Hier besteht ein Wendepunkt, aber wiederum keine Unstetigkeitstelle. Von diesem Wendepunkt ausgehend nimmt der Durchmesser der Folie wieder zu, bis zum Vestibulärring.

Auch wenn andere Kurvenformen der Folie insofern möglich sind, ist die Realisierung einer Kettenlinie oder eines V mit abgerundeter Spitze bevorzugt.

Weitere Vorteile, Einzelheiten und Merkmale der Erfindung ergeben sich aus der nachfolgenden Beschreibung mehrerer Ausführungsbeispiele anhand der beigefügten Zeichnungen.

Es zeigen:
- Fig. 1: eine schematische dreidimensionale Liniendarstellung einer Ausführungsform eines erfindungsgemäßen Folienspannelements;
- Fig. 2A, 2B und 2C: je eine Schnittdarstellung von 3 Ausführungsformen eines erfindungsgemäßen Folienspannelements; und
- Fig. 3: eine Seitenansicht einer weiteren Ausführungsform eines erfindungsgemäßen Folienspannelements.

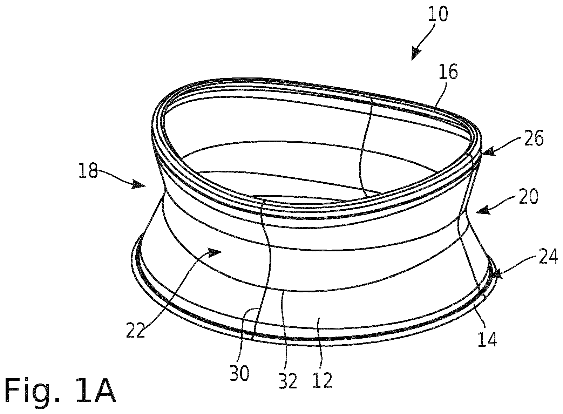

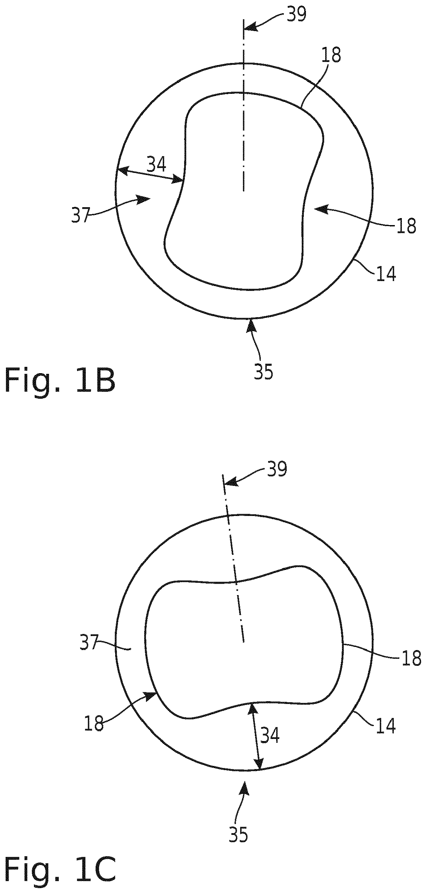

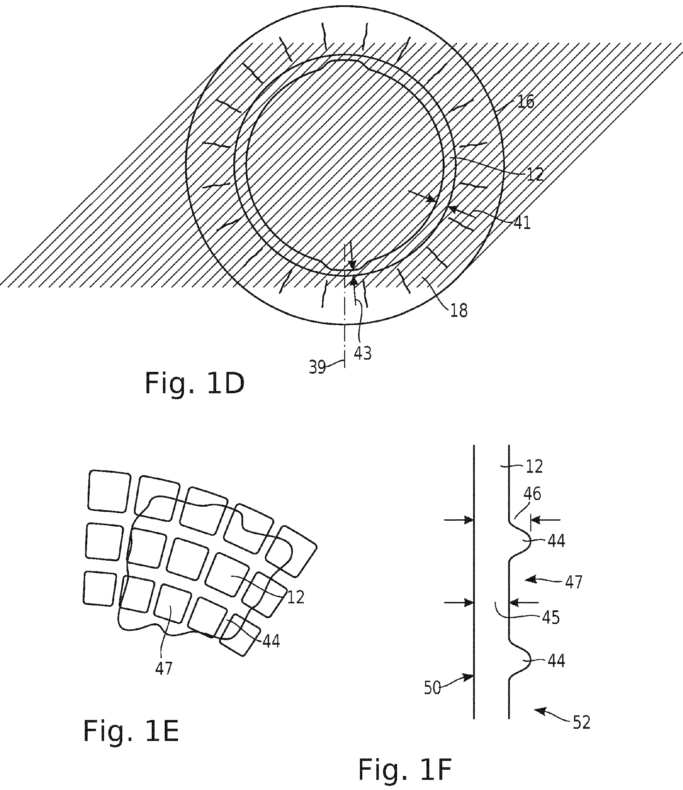

Fig. 1 zeigt ein Folienspannelement 10, das in an sich bekannter Weise aus einer Folie 12, einem Lippenring 14 und einem Vestibulärring 16 besteht. Das Folienspannelement 10 insgesamt ist ringförmig, wobei der grundsätzliche Aufbau im Wesentlichen einem Kreisring entspricht, aber in einer modifizierten Ausführungsform auch eine andere Ringform, wie beispielsweise eine ovale oder elliptische Form aufweisen kann.

Im dem dargestellten Ausführungsbeispiel weist der Vestibulärring 16 in ebenfalls an sich bekannter Weise einen etwas kleineren Durchmesser als der Lippenring 14 auf.

Erfindungsgemäß ist die Folie 12 zwischen den beiden Ringen 14 und 16 eingeschnürt.

Im dargestellten Ausführungsbeispiel ist diese Einschnürung 18 symmetrisch ausgebildet, so dass der tiefste Punkt der Einschnürung 18 in der Mitte zwischen Lippenring 14 und Vestibulärring 16 ist.

Die Einschnürung 18 ist in dem dargestellten Ausführungsbeispiel ringförmig umlaufend ausgebildet, so dass also an allen Stellen des ringförmigen Folienspannelements 10 der geringste Durchmesser des Folienspannelements im Bereich der Einschnürung 18 ist.

In dem in Fig. 1 dargestellten Ausführungsbeispiel verläuft die Einschnürung 18 im Wesentlichen V-förmig. Der tiefste Punkt des V 20 der Einschnürung 18 ist mit einem Radius 22 versehen, der in dem dargestellten Ausführungsbeispiel kleiner als die Tiefe der Einschnürung 18 ist.

Die in Fig. 1 dargestellte Position und Form des Folienspannelements 10 ergibt sich, wenn das Folienspannelement 10 spannungsfrei ist. Wenn beispielsweise der Lippenring 14 auf eine Unterlage gelegt wird und das Folienspannelement 10 am Vestibulärring 16 angehoben wird, bis sich die in Fig. 1 dargestellte Form ergibt, ist die Folie 12 spannungsfrei (abgesehen von ihren sehr geringen Eigengewicht). In dieser Form ist die Folie 12 faltenfrei oder nahezu faltenfrei und die Einschnürung 18 stellt sich als besondere vorgeformte Form der Folie 12 dar.

Das Folienspannelement 10 lässt sich beispielsweise in einem Spritzgießprozess herstellen. Bevorzugt sind dann der Lippenring 14 und der Vestibulärring 16 in die Spritzgussform eingelegt und werden von dem Material der Folie 12 umspritzt. Das Material der Folie 12 ist in an sich bekannter Weise elastisch und vergleichsweise weich, während die Ringe 14 und 16 aus einem demgegenüber härteren Kunststoffmaterial bestehen.

Die Spritzgießform ist so ausgebildet, dass sich die Stärke der Folie 12 von den Ringen 14 und 16 ausgehend kontinuierlich und in einem sanften Übergang verjüngt. Der Bereich der Verjüngung kann gering gehalten sein, beispielsweise etwa der Stärke jedes Ringes 14 und 16 entsprechen. Durch diese Lösung wird mit geringem Materialaufwand ein hinsichtlich der Belastbarkeit optimierter Aufbau der Folie 12 möglich.

Ein Standard-OptraGate-Folienspannelement hätte in der Darstellung gemäß Fig. 1 einen konischen Aufbau ohne Einschnürung 18.

Die Belastbarkeit des erfindungsgemäßen Folienspannelements 10 ist mindestens gleich gut. Der Materialaufwand ist aber etwas geringer, etwa um 10 ist bis 20 %, je nach Tiefe der Einschnürung 18. Durch die Durchmesserreduktion im Bereich der Einschnürung 18 ist weniger Material erforderlich.

Im Vergleich mit dem ebenfalls bekannten Folienspannelement 10 gemäß der vorstehend genannten Patentveröffentlichung, die ein aufgedoppeltes Elastikband zeigt, ist der Materialaufwand deutlich geringer, da kein Elastikband verwendet wird, sondern die Folie 12 über ihre Erstreckung zwischen den Ringen 14 und 16 - abgesehen ebenfalls von der vorstehend erwähnten Verjüngung 24 - eine konstante und gleich bleibende Stärke aufweist.

Überraschend ergibt sich durch die zentral vorgesehene Einschnürung 18, die bevorzugt V-förmig ist, eine ausgesprochen tragefreundliche Anpassung an die Lippen des Patienten die den Lippendruck reduziert und damit die Akzeptanz erhöht.

Überraschend lässt sich durch den Zentralradius 22 insofern eine deutliche Verbesserung im Vergleich mit einem asymmetrisch angeordneten Elastikband erzielen.

Wesentlicher Vorteil der Erfindung ist, dass durch entsprechende Werkzeuggestaltung, z.B. der Spritzgussform, die Einschnürung zentral, aber eben auch nicht-zentral liegen kann, also z.B. partiell näher an einem der beiden Ringe oder ggf. mit unterschiedlichen V-Tiefen. Sie kann auch insgesamt nur partiell vorhanden sein.

Da die Stärke der Folie 12 im Wesentlichen gleichbleibend über ihre gesamte Erstreckung zwischen den beiden Ringen 14 und 16 ist, werden eingeleitete Spannungen gleichmäßig verteilt. Die Belastbarkeit ist insofern erfindungsgemäß ebenfalls überraschend im Vergleich mit der Elastikband-Lösung trotz deren höherem Materialaufwand verbessert.

Klarstellunghalber sei angemerkt, dass die in Fig. 1 zusätzlich dargestellten Linien, beispielsweise Linien 30 und 32, reine Formlinien sind, die die Form des Folienspannelements 10 deutlich machen sollen, aber auf dem tatsächlichen Folienspannelement 10 nicht vorhanden sind. Vielmehr erstreckt sich die Folie 12 tatsächlich glatt und ohne Auflagen und Übergänge zwischen den Ringen 14 und 16, aber auch entlang ihrer Ringform.

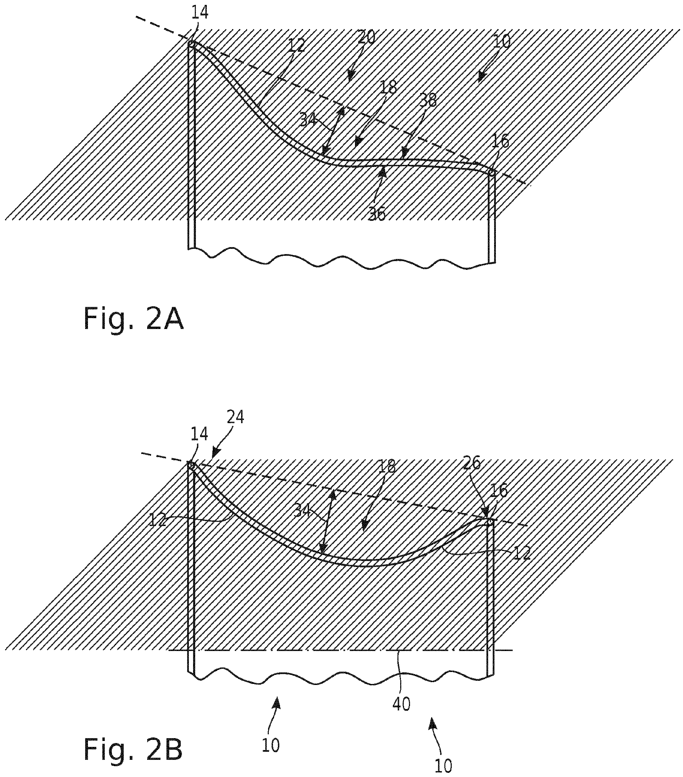

Aus Fig. 2A und aus Fig. 2B sind zwei unterschiedliche Formen von Folienspannelementen 10 im Schnitt ersichtlich.

Fig. 2A zeigt ein Folienspannelement 10 mit einer Folie 12, die sich mit einer Einschnürung 18 im Wesentlichen in Form eines V 20 zwischen den Ringen 14 und 16 erstreckt. Der tiefste Punkt des V 20 liegt in dem dargestellten Ausführungsbeispiel in der Mitte zwischen den Ringen 14 und 16. Die Tiefe 34 der Einschnürung 18 beträgt im dargestellten Ausführungsbeispiel mehr als die 15-fache Stärke der Folie 12. Mit "Stärke" ist die Erstreckung der Folie 12 zwischen ihrer radial einwärts weisenden Innenfläche 36 und ihrer radial auswärts weisenden Außenfläche 38 gemeint. Innerhalb dieser Stärke erstreckt sich die Folie 12 homogen aus einem einheitlichen Material.

In dem dargestellten Ausführungsbeispiel ist die Stärke der Folie 12 zwischen den Ringen 14 und 16 konstant, gegebenenfalls abgesehen von den zugehörigen Verjüngungen 24 und 26. In dem Ausführungsbeispiel gemäß Fig. 2A ist jedoch keine Verjüngung 24 und 26 vorgesehen; vielmehr ist bei diesem Ausführungsbeispiel jeder Ring 14 und 16 so abgeflacht, dass sich die Folie 12 anschließend an den Ring mit im wesentlichen gleich bleibender Stärke von diesem wegerstreckt.

Demgegenüber ist die Stärke der Ringe 14 und 16 sowohl bei der Ausführungsform gemäß Fig.1 als auch bei der Ausführungsform gemäß Fig. 2B größer als die Stärke der Folie 12, so dass bevorzugt Verjüngungen 24 und 26 vorgesehen sind.

In dem dargestellten Ausführungsbeispiel gemäß Fig. 2A beträgt die Stärke der Folie 12 0,4 mm.

Es versteht sich, dass auch beliebige andere an die Größe und Form der Folienspannelemente 10 angepasste Folienstärken möglich sind, beispielsweise zwischen 0,1 mm für kleine Folienspannelemente für Kindermünder und 1 mm bei starker Belastung oder bei Anwendungen an größeren Mündern bei welchen derartige Folienspannelemente 10 ebenfalls verwendet werden können.

Wie aus Fig. 2A ersichtlich ist, ist die Länge der Ringe 14 und 16 entlang der Erstreckung des Folienspannelementes 10 in Richtung der Folie 12 gegenüber der radialen Erstreckung vergrößert. Diese Form führt zu einer erhöhten Stabilität gegen eine axiale Durchwölbung, aber dennoch eine gute Anpassbarkeit an die Mundform des Patienten, insbesondere im Bereich des Vestibulärrings 16.

Aus Fig. 2B ist eine modifizierte Ausführungsform des erfindungsgemäßen Folienspannelements 10 ersichtlich. Bei dieser Ausführungsform erstreckt sich die Folie 12 mit der Einschnürung 18 nach der Art einer Kettenlinie durchgebogen zwischen den Ringen 14 und 16. Die Ringe 14 und 16 haben im Wesentlichen einen kreisförmigen Aufbau im Querschnitt und eine Stärke von 1,2 mm. Die etwas übertrieben dargestellte Stärke der Folie 12 beträgt bei dieser Ausführungsform 0,3 mm. Auch hier ist die Stärke gleichbleibend und frei von Auflagen und Einlagen über die gesamte Erstreckung der Folie 12 zwischen den Ringen 14 und 16.

In Fig. 2B ist zusätzlich eine gedachte Achse 40 des Folienspannelements 10 eingezeichnet. Diese besteht auch bei den übrigen Ausführungsformen des Folienspannelements 10, auch wenn sie dort nicht eingezeichnet ist. Sie macht deutlich, in welcher Richtung eine radiale Erstreckung oder ein radiales "Weisen" gedacht ist.

In dem Ausführungsbeispiel gemäß Fig. 2B beträgt die Tiefe 34 der Einschnürung 18 das 30-fache der Stärke der Folie 12.

Auch wenn hier die tiefste Stelle der Einschnürung 18 sich je in der Mitte zwischen den Ringen 14 und 16 erstreckt, versteht es sich, dass eine leichte Abweichung hiervon entweder in Richtung Vestibulärring 16 oder in Richtung Lippenring 14 möglich ist, ohne den Bereich der Erfindung zu verlassen.

In einer modifizierten Ausführungsform ist es vorgesehen, dass die Einschnürung 18 an der für die Wangen bestimmten Stelle stärker ist.

In einer alternativen Ausführungsform ist es vorgesehen, dass die Tiefe 34 der Einschnürung 18 an der für die Mundmitte bestimmten Stelle stärker ist. Damit ist sie im Bereich der sagittalen Ebene des Patienten am stärksten.

Auch wenn hier die Einschnürung 18 mit konstanter Tiefe 34 über den gesamten ringförmigen Verlauf der Folie 12 - also in Umfangsrichtung oder in tangentialer Richtung - vorgesehen ist, ist es in einer weiteren Ausführungsform vorgesehen, die Tiefe 34 der Einschnürung 18 über die Ringform zu variieren. Die Einschnürung 18 kann sich auch lediglich teilringförmig erstrecken.

Der Bereich der Einschnürung 18 muss sich auch nicht über den gesamten Abstand zwischen Vestibulärring 16 und Lippenring 14 erstrecken; es ist auch möglich, die Einschnürung 18 beispielsweise zentral mit einer geringeren achsparallelen Erstreckung vorzusehen.

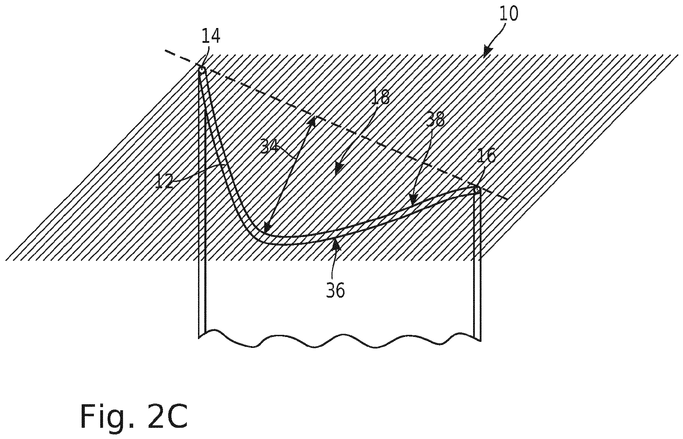

Fig. 2C zeigt eine weitere Ausführungsform eines erfindungsgemäßen Folienspannelements 10. Bei dieser Ausführungsform ist die Einschnürung 18 zum einen deutlich tiefer, Die Tiefe 34 ist mehr als die Hälfte des Abstands zwischen dem Lippenring 14 und dem Vestibulärring 16. Bei den Ausführungsformen gemäß den Fig. 2A und 2B beträgt die Tiefe 34 demgegenüber nur etwa 15 % des Abstands zwischen dem Lippenring 14 und dem Vestibulärring 16.

Erfindungsgemäß kann die Tiefe 34 in weiten Bereichen an die Erfordernisse angepasst werden, z.B. durch entsprechende Ausgestaltung der Spritzgussform. Die Tiefe 34 kann erfindungsgemäß bevorzugt zwischen 8% und 80% des Abstands zwischen dem Lippenring 14 und dem Vestibulärring 16. betragen.

Zum anderen ist die Einschnürung mit ihrem Schwerpunkt bei der Ausführungsform gemäß Fig. 2C deutlich näher an dem Lippenring 14 als an dem Vestibulärring 16. Der Lippenring 14 ist größer als der Vestibulärring 16. Dadurch, dass der Durchmesser der Folie 12 vom Lippenring 14 ausgehend stark abfällt, z.B. auf die Hälfte des Durchmessers des Lippenrings 14 innerhalb eines Viertels des Abstands zwischen dem Lippenring 14 und dem Vestibulärring 16.

Durch diesen starken Durchmesserabfall wird Folienmaterial in erheblichem Umfang eingespart.

In den dargestellten Ausführungsformen gemäß den Fig. 1, 2A, 2B und 2C beträgt der axiale Bereich der Einschnürung 18 99 % bei Fig. 1 und 2B und 100 % bei Fig. 2A und 2C.

Wie anhand von Fig. 1 bereits erläutert, ist es bevorzugt, dass die Folie 12 spannungsfrei die in den Figuren dargestellte Form hat. Durch die Einschnürung 18 ist sie insofern gleichsam radial einwärts weisend spannungsfrei geformt oder vorgeformt.

Alternativ zum Spritzgießprozess ist es auch möglich, dass erfindungsgemäße Folienspannelement 10 mittels eines Pressprozesses, eines Tauchprozesses oder eines Glasformprozesses herzustellen.

Wenn die erfindungsgemäße Folie aus Silikonmaterialien hergestellt wird, können die Ringe zuvor in die Pressform eingelegt und umpresst werden.

Die Folie 12 kann auch über den Umfang betrachtet unterschiedliche Stärken aufweisen. Diese unterschiedlichen Stärken korrespondieren mit den unterschiedlichen Tiefen 34 der Einschnürung 18.

In achsparalleler Richtung zur Achse 40 ist die Stärke der Folie 12 bevorzugt allerdings stets gleichbleibend Stärke zwischen Lippenring 14 und Vestibulärring 16, abgesehen gegebenenfalls von Verjüngungen 24 und 26.

In einer weiteren modifizierten Ausgestaltung ist es vorgesehen, dass die Folie 12 eine Netzstruktur hat. Diese ist bevorzugt nach der FEM-Methode berechnet und dient dazu, die Materialeinsparung noch zu optimieren. Im Bereich der Maschen des Netzes trägt dann die Stärke der Folie 12 beispielsweise lediglich 0,1 mm und im Bereich des Gitters des Netzes 0,3 mm.

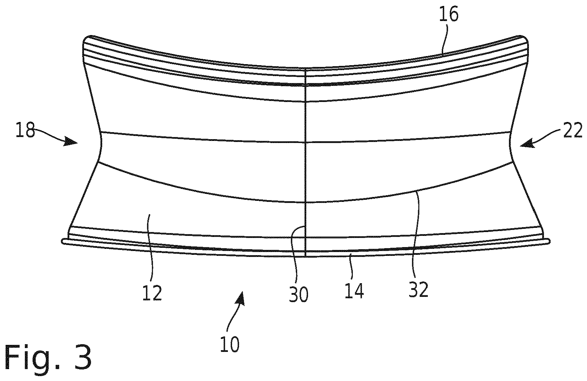

Aus Fig. 3 ist eine andere Ansicht und eine gegenüber der Ausführungsform gemäß Fig.1 etwas modifizierten Ausführungsform des Folienspannelements 10 dargestellt. Gleiche Bezugszeichen weisen hier wie auch in den anderen Figuren auf gleiche oder entsprechende Teile hin.

Bei der Ausführungsform gemäß Fig. 3 sind der Vestibulärring 16 und der Lippenring 14 in der dargestellten Seitenansicht gebogen, der Vestibulärring 16 etwas stärker als der Lippenring 14.

Bei dieser Ausführungsform sind entsprechend Fig. 1 Verjüngungen 26 und 24 vorgesehen. Die Ringe 14 und 16 sind von dem Material der Folie 12 umspritzt.

Durch die gebogene Form lässt sich der Vestibulärring 16 leichter in den Mund des Patienten einführen und nimmt auch leichter die gewünschte ovale Form an, die dem Mund des Patienten entspricht.

## Patentansprüche

1. Folienspannelement, insbesondere für die Vornahme dentaler Bearbeitungen im Mundraum eines Patienten, mit einem Vestibulärring (16) und einem Lippenring (14), zwischen welchen und ggf. über welche hinaus sich eine Folie (12) erstreckt, die von den Ringen (14,16) aufgespannt gehalten ist, wobei die Folie und die Ringe elastisch verformbar sind, **dadurch gekennzeichnet, dass** die Folie (12) im Verlauf zwischen Lippenring (14) und Vestibulärring (16) mindestens in einem Bereich eine sich radial einwärts erstreckende Einschnürung (18) aufweist, mindestens in welchem Bereich die Folie (12) sich spannungsfrei und einstückig zwischen ihrer Innenfläche (36) und Außenfläche (38) erstreckt insbesondere frei von Auflagen und/oder Einlagen.

2. Folienspannelement nach Anspruch 1, **dadurch gekennzeichnet, dass** die Folie (12) in dem Bereich zwischen Lippenring (14) und Vestibulärring (16) eine im Wesentlichen, also +/- 20 %, konstante, insbesondere konstante Stärke aufweist und/oder dass die Folie (12) an der Einschnürung (18) einen geringeren Durchmesser als an den Ringen (14,16) hat.

3. Folienspannelement nach einem der vorhergehenden Ansprüche, **dadurch gekennzeichnet, dass** der Bereich der Einschnürung (18) ringförmig oder teilringförmig ist.

4. Folienspannelement nach einem der vorhergehenden Ansprüche, **dadurch gekennzeichnet, dass** der Bereich der Einschnürung (18) sich über weniger als 99% und mehr als 30% des Abstandes zwischen dem Vestibulärring (16) und dem Lippenring (14) erstreckt.

5. Folienspannelement nach einem der vorhergehenden Ansprüche, **dadurch gekennzeichnet, dass** die Tiefe (34) der Einschnürung (18), insbesondere radial einwärts betrachtet, um den Umfang der Folie (12) unterschiedlich ist

6. Folienspannelement nach Anspruch 5, **dadurch gekennzeichnet, dass** die Tiefe (34) der Einschnürung (18) an der für die Wangen bestimmten Stelle stärker ist.

7. Folienspannelement nach Anspruch 5, **dadurch gekennzeichnet, dass** die Tiefe (34) der Einschnürung (18) an der für die Mundmitte, also an der sagittalen Ebene und um diese herum, bestimmten Stelle stärker ist.

8. Folienspannelement nach einem der vorhergehenden Ansprüche, **dadurch gekennzeichnet, dass** die Einschnürung (18) die Folie (12) radial einwärts spannungsfrei formt und/oder die Stelle der größten Tiefe (34) der Einschnürung in der Mitte zwischen den Ringen (14,16) oder zum Lippenring (14) hin gegenüber der Mitte verlagert ist.

9. Folienspannelement nach einem der vorhergehenden Ansprüche, **dadurch gekennzeichnet, dass** die Folie (12) sich, im Schnitt durch Lippenring (14) und Vestibulärring (16) betrachtet, im biegeschlaffen Zustand nach der Art einer Kettenlinie erstreckt, oder, dass die Folie (12) sich - im Längsschnitt parallel zu einer Achse (40) der Folienspannelements (10) betrachtet - im wesentlichen nach der Art eines V (20) erstreckt.

10. Folienspannelement nach einem der vorhergehenden Ansprüche, **dadurch gekennzeichnet, dass** die Folie (12) durch einen Spritzgießprozess, einen Pressprozess, einen Tauchprozess oder einen Blasformprozess hergestellt ist.

11. Folienspannelement nach einem der vorhergehenden Ansprüche, **dadurch gekennzeichnet, dass** die Folie (12) zwischen dem Lippenring (14) und dem Vestibulärring (16), um ihren Umfang betrachtet, unterschiedliche Stärken aufweist.

12. Folienspannelement nach einem der vorhergehenden Ansprüche, **dadurch gekennzeichnet, dass** die Folie (12) die Einschnürung (18) eine maximale Tiefe (34) von mindestens dem 5-fachen der maximalen Stärke der Folie (12) .hat, insbesonders von mindestens dem 10-fachen und besonders bevorzugt von mindestens dem 15-fachen.

13. Folienspannelement nach einem der vorhergehenden Ansprüche, **dadurch gekennzeichnet, dass** die Folie (12) an der Einschnürung (18) um Ihren Umfang betrachtet unterschiedliche Stärken, also Wanddicken, aufweist.

14. Folienspannelement nach einem der vorhergehenden Ansprüche, **dadurch gekennzeichnet, dass** die Folie (12) eine Netzstruktur aufweist, die insbesondere unter Berücksichtigung einer Berechnung nach der FEM festgelegt ist.

15. Folienspannelement nach Anspruch 14, **dadurch gekennzeichnet, dass** die Maschen des Netzes eine geringere Wandstärke als das Gitter des Netzes aufweisen.

## Claims

1. A film tensioning element, in particular for performing dental operations in a patient's oral cavity, comprising a vestibular ring (16) and a lip ring (14) between which and optionally beyond which a film (12) extends, said film being held tensioned by the rings (14, 16), the film and the rings being resiliently deformable, **characterised in that** the film (12) has a radially inwardly extending constriction (18), at least in a region, in the course between the lip ring (14) and vestibular ring (16), the film (12), at least in said region, extending without tension and in a single piece between the inner face (36) and outer face (38) thereof, in particular without onlays and/or inlays.

2. The film tensioning element according to claim 1, **characterised in that** the film (12) has, in the region between the lip ring (14) and vestibular ring (16), a substantially, i.e. +/- 20 %, constant, in particular constant thickness, and/or **in that** the film (12) has a smaller diameter at the constriction (18) than at the rings (14, 16) .

3. The film tensioning element according to any of the preceding claims, **characterised in that** the region of the constriction (18) is ring-shaped or part-ring-shaped.

4. The film tensioning element according to any of the preceding claims, **characterised in that** the region of the constriction (18) extends over less than 99 % and more than 30 % of the distance between the vestibular ring (16) and the lip ring (14).

5. The film tensioning element according to any of the preceding claims, **characterised in that** the depth (34) of the constriction (18), in particular as viewed radially inwards, is variable over the periphery of the film (12).

6. The film tensioning element according to claim 5, **characterised in that** the depth (34) of the constriction (18) is greater at the point intended for the cheeks.

7. The film tensioning element according to claim 5, **characterised in that** the depth (34) of the constriction (18) is greater at the point intended for the mouth centre, in other words at and around the sagittal plane.

8. The film tensioning element according to any of the preceding claims, **characterised in that** the constriction (18) shapes the film (12) radially inwards without tension and/or the point of the greatest depth (34) of the constriction is in the centre between the rings (14, 16) or shifted towards the lip ring (14) with respect to the centre.

9. The film tensioning element according to any of the preceding claims, **characterised in that** the film (12), as viewed in a section through the lip ring (14) and vestibular ring (16), extends in the manner of a catenary in the limp state, or **in that** the film (12), as viewed in a longitudinal section parallel to an axis (40) of the film tensioning element (10), extends substantially in the manner of a V (20).

10. The film tensioning element according to any of the preceding claims, **characterised in that** the film (12) is manufactured by an injection-moulding process, a pressing process, a dipping process or a blow-moulding process.

11. The film tensioning element according to any of the preceding claims, **characterised in that** the film (12) has different thicknesses, as viewed about the periphery thereof, between the lip ring (14) and vestibular ring (16).

12. The film tensioning element according to any of the preceding claims, **characterised in that** at the constriction (18) the film (12) has a maximum depth (34) of at least 5 times, in particular at least 10 times and particularly preferably at least 15 times the maximum thickness of the film (12).

13. The film tensioning element according to any of the preceding claims, **characterised in that** at the constriction (18) the film (12) has different thicknesses, in other words wall thicknesses, as viewed about the periphery thereof.

14. The film tensioning element according to any of the preceding claims, **characterised in that** the film (12) has a net structure, which is established in particular while taking into account an FEM calculation.

15. The film tensioning element according to claim 14, **characterised in that** the meshes of the net have a lower wall thickness than the lattice of the net.

## Revendications

1. Élément tendeur de film, en particulier pour effectuer des traitements dentaires dans la cavité buccale d'un patient, comprenant une bague vestibulaire (16) et une bague labiale (14), entre lesquels et éventuellement au-delà desquels s'étend un film (12) qui est maintenu tendu par les bagues (14, 16), où le film et les bagues sont déformables élastiquement, **caractérisé en ce que** dans le tracé entre la bague labiale (14) et la bague vestibulaire (16), le film (12) présente au moins dans une zone, un rétrécissement (18) qui s'étend radialement vers l'intérieur, au moins dans cette zone le film (12) s'étend sans tension et d'un seul tenant entre sa surface intérieure (36) et sa surface extérieure (38), en particulier sans appuis et/ou garnitures.

2. Élément tendeur de film selon la revendication 1, **caractérisé en ce que** le film (12) présente dans la zone entre la bague labiale (14) et la bague vestibulaire (16) une épaisseur essentiellement, c'est à dire +/- 20%, constante, en particulier constante, et/ou **en ce que** le film (12) présente un diamètre plus petit au niveau du rétrécissement (18) qu'au niveau des bagues (14, 16).

3. Élément tendeur de film selon l'une des revendications précédentes, **caractérisé en ce que** la zone du rétrécissement (18) est de forme annulaire ou partiellement annulaire.

4. Élément tendeur de film selon l'une des revendications précédentes, **caractérisé en ce que** la zone du rétrécissement (18) s'étend sur moins de 99% et plus de 30% de la distance entre la bague vestibulaire (16) et la bague labiale (14).

5. Élément tendeur de film selon l'une des revendications précédentes, **caractérisé en ce que** la profondeur (34) du rétrécissement (18), en particulier vu radialement vers l'intérieur, est différente autour de la circonférence du film (12).

6. Élément tendeur de film selon la revendication 5, **caractérisé en ce que** la profondeur (34) du rétrécissement (18) est plus importante à l'endroit destiné aux joues.

7. Élément tendeur de film selon la revendication 5, **caractérisé en ce que** la profondeur (34) du rétrécissement (18) est plus importante à l'endroit destiné au milieu de la bouche, c'est-à-dire dans le plan sagittal et autour de celui-ci.

8. Élément tendeur de film selon l'une des revendications précédentes, **caractérisé en ce que** le rétrécissement (18) forme le film (12) sans tension radialement vers l'intérieur et/ou **en ce que** l'endroit de la plus grande profondeur (34) du rétrécissement est décalé par rapport au centre entre les bagues (14, 16) ou vers la bague labiale (14).

9. Élément tendeur de film selon l'une des revendications précédentes, **caractérisé en ce que** le film (12), vu en coupe à travers la bague labiale (14) et la bague vestibulaire (16), s'étend à l'état relâché en flexion à la manière d'une ligne de chaîne, ou **en ce que** le film (12) - vu en coupe longitudinale parallèlement à un axe (40) de l'élément tendeur de film (10) - s'étend essentiellement à la manière d'un V (20).

10. Élément tendeur de film selon l'une des revendications précédentes, **caractérisé en ce que** le film (12) est fabriqué par un processus de moulage par injection, un processus de pressage, un processus d'immersion ou un processus de soufflage.

11. Élément de serrage en film selon l'une des revendications précédentes, **caractérisé en ce que** le film (12) présente des épaisseurs différentes entre la bague labiale (14) et la bague vestibulaire (16), vu autour de sa circonférence.

12. Élément tendeur de film selon l'une quelconque des revendications précédentes, **caractérisé en ce que** le film (12) présente le rétrécissement (18) d'une profondeur maximale (34) d'au moins 5 fois l'épaisseur maximale du film (12), en particulier d'au moins 10 fois et de manière particulièrement préférée d'au moins 15 fois.

13. Élément tendeur de film selon l'une des revendications précédentes, **caractérisé en ce que** le film (12) présente des épaisseurs, c'est à dire des épaisseurs de paroi, différentes, au niveau du rétrécissement (18), vu sur sa circonférence.

14. Élément tendeur de film selon l'une des revendications précédentes, **caractérisé en ce que** le film (12) présente une structure réticulée qui est déterminée en particulier en tenant compte d'un calcul selon la méthode des éléments finis (MEF).

15. Élément de tension de film selon la revendication 14, **caractérisé en ce que** les mailles du filet présentent une épaisseur de paroi inférieure à celle du treillis du filet.
